Europäisches Patentamt
European Patent Office
Office européen des brevets

(19)

(11) Publication number: **0 006 961**
**A1**

# (12) EUROPEAN PATENT APPLICATION

(21) Application number: 79100406.2

(22) Date of filing: 12.02.79

(51) Int. Cl.³: C 07 C 91/30
C 07 C 93/14, C 07 D 295/08
A 61 K 31/135, C 07 C 93/26

(30) Priority: 21.02.78 CH 1845/78

(43) Date of publication of application:
23.01.80 Bulletin 80/2

(84) Designated Contracting States:
BE CH DE FR GB LU NL SE

(71) Applicant: SANDOZ LTD.
Lichtstrasse 35
CH-4002 Basel(CH)

(72) Inventor: Haefliger, Walter, Dr.
Marignanostrasse 37
CH-4059 Basle(CH)

(72) Inventor: Closse, Annemarie, Dr.
Schafmattweg 78
CH-4102 Binningen(CH)

## (54) New phenylethylamines, process for their preparation, and pharmaceutical compositions containing them.

(57) Phenethylamine compounds useful as anti-phlogistic, anti-arthritic, anti-pyretic and analgesic agents, of formula I

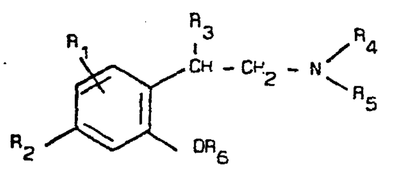

(I)

wherein $R_1$ is hydrogen, halogen, hydroxy, nitro, $(C_{1-4})$ alkyl, $(C_{1-4})$alkoxy, $(C_{1-4})$alkylthio, lower acyloxy or lower acylamino, $R_2$ is $(C_{3-8})$cycloalkyl or a phenyl radical, $R_3$ is hydrogen or $(C_{1-4})$alkyl, $R_4$ and $R_5$ are the same or different and are hydrogen, $(C_{1-4})$alkyl or $(C_{7-11})$phenylalkyl, or $R_4$ and $R_5$, together with the nitrogen atom to which they are bound, form a 1-pyrrolidinyl, piperidino or morpholino radical, or $R_4$ and $R_6$ together form a hydrolysable methylene moiety and $R_6$ is hydrogen or a hydrolysable acyl radical, and a process for their production which comprises reducing a compound of formula II

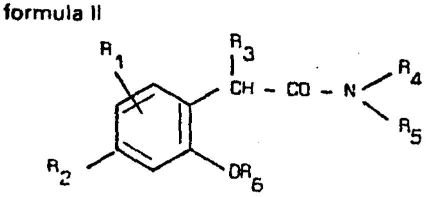

(II)

in which $R_1$ to $R_6$ are as defined above, and where required appropriately acylating any resulting product having a free hydroxy or amino group and/or condensing any resulting compound wherein $R_4$ and $R_5$ are hydrogen with an appropriate aldehyde or ketone.

EP 0 006 961 A1

New phenylethylamines, process for their preparation, and pharmaceutical compositions containing them

The present invention relates to new phenylethylamines, their preparation and pharmaceutical compositions containing them.

In accordance with the invention there are provided new compounds of formula I

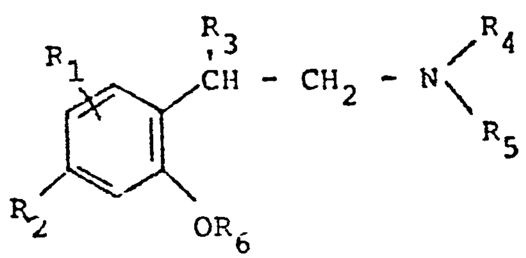

(I)

wherein

$R_1$ is hydrogen, halogen, hydroxy, nitro, $(C_{1-4})$alkyl, $(C_{1-4})$alkoxy, $(C_{1-4})$alkylthio, lower acyloxy or lower acylamino,

$R_2$ is $(C_{3-8})$cycloalkyl or a phenyl radical,

$R_3$ is hydrogen or $(C_{1-4})$alkyl,

$R_4$ and $R_5$ are the same or different and are hydrogen, $(C_{1-4})$alkyl or $(C_{7-11})$phenylalkyl, or

$R_4$ and $R_5$, together with the nitrogen atom to which they are bound, form a 1-pyrrolidinyl, piperidino, or morpholino radical, or

$R_4$ and $R_5$ together form a hydrolysable methylene moiety and

$R_6$ is hydrogen or a hydrolysable acyl radical.

Any alkyl, alkoxy or alkylthio radical mentioned herein has preferably 1-2 carbon atoms or especially one carbon atom. Halogen means fluorine, chlorine, bromine or iodine.

When $R_1$ is halogen, this is preferably chlorine or bromine or especially chlorine.

When $R_1$ is lower acyloxy or lower acylamino, the acyl moiety may be derived from an aliphatic carboxylic acid containing up to 4 carbon atoms, preferably 2 carbon atoms.

$R_1$ is preferably halogen or alkyl. $R_1$ is conveniently para with respect to the group $OR_6$.

When $R_2$ is cycloalkyl, this is preferably cyclopentyl or cyclohexyl, especially cyclohexyl. When $R_2$ is a phenyl radical this is conveniently an unsubstituted phenyl radical or a phenyl radical containing one or more substituents selected from halogen, hydroxy, nitro, amino, trifluoromethyl, $(C_{1-4})$alkyl and $(C_{1-4})$alkoxy. Preferably only one

substituent is present, conveniently in the para position of the phenyl radical. Preferably the substituent is selected from halogen, preferably chlorine or fluorine, alkyl or alkoxy.

$R_2$ is preferably a phenyl radical. especially an unsubstituted phenyl radical.

Preferably $R_3$ is hydrogen.

When $R_4$ or $R_5$ is phenylalkyl, this preferably contains 7 or 8 carbon atoms or especially 7 carbon atoms.

Conveniently, $R_4$ and $R_5$ are the same or different and are hydrogen, $(C_{1-4})$alkyl or $(C_{7-11})$ phenylalkyl, or together with the nitrogen atom to which they are bound, form a 1-pyrrolidinyl, piperidino, or morpholino radical.

$R_4$ and $R_5$ are preferably the same, and are each preferably alkyl or more preferably hydrogen. When $R_4$ and $R_5$ are different, $R_5$ is desirably hydrogen.

When $R_4$ and $R_5$ together are a hydrolysable methylene moiety, this is a physiologically acceptable hydrolysable moiety, which may be substituted, e.g. alkylidene, cycloalkylidene or arylalkylidene. It is to be appreciated that the compound may be a compound of formula I wherein $R_4$ and $R_5$ are each hydrogen in the form of a Schiff's base. Thus the radical may be

derived from a non-toxic aldehyde or ketone and may for example have the formula

$$=CR_7R_8$$

wherein $R_7$ and $R_8$ are the same or different and are hydrogen, $(C_{1-4})$alkyl, a phenyl radical or a phenylalkyl radical, or $R_7$ and $R_8$ together are $(C_{3-6})$alkylene or $(C_{3-6})$alkenylene.

The phenylalkyl radical may be phenylalkyl of 7 to 11 carbon atoms (ignoring any carbon containing substituents), unsubstituted or substituted in the phenyl ring. When the phenyl radical or the phenylalkyl radical is substituted in the phenyl ring, there is preferably one substituent, e.g. halogen of atomic number from 9 to 35, hydroxy, nitro, amino, trifluoromethyl, $(C_{1-4})$alkyl or $(C_{1-4})$alkoxy.

Conveniently $R_7$ and $R_8$ are each alkyl. Alternatively $R_7$ is hydrogen and $R_8$ is for example alkyl or a phenyl radical such as phenyl or o-hydroxyphenyl.

When $R_6$ is a hydrolysable acyl radical, this is an acyl group which is hydrolyzed under physiological conditions to form a non-toxic acid and may be for example of formula $-COR_9$ wherein

$R_9$ is alkyl of 1 to 12 carbon atoms, cycloalkyl of 3 to 7 carbon atoms, phenyl, phenylalkyl of 7 to 12 carbon atoms, phenyl or phenylalkyl of 7 to 12 carbon atoms monosubstituted in the phenyl ring by alkyl of 1 to 4 carbon atoms, or mono- or independently disubstituted in the phenyl ring by halogen of atomic number of from 9 to 35, or mono- or independently di- or independently trisubstituted in the phenyl ring by alkoxy of 1 to 4 carbon atoms. $R_6$ is preferably hydrogen.

In a group of compounds $R_1$ is para to the group $OR_6$ and $R_1$ is hydrogen, methyl or halogen, $R_2$ is cycloalkyl, phenyl or phenyl substituted by halogen, alkyl or alkoxy, preferably phenyl, $R_4$ and $R_5$ are the same or different and are hydrogen, $(C_{1-4})$alkyl or $(C_{7-11})$phenylalkyl or $R_4$ and $R_5$, together with the nitrogen atom to which they are bound, form a 1-pyrrolidinyl, piperidino or morpholino radical and $R_6$ is hydrogen.

The present invention provides a process for the production of a compound of formula I as defined above, which comprises reducing a compound of formula II

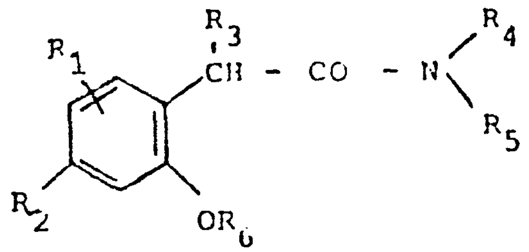

(II)

in which $R_1$ to $R_6$ are as defined above and where required appropriately acylating any resulting product having a free hydroxy group and/or condensing any resulting compound wherein $R_4$ and $R_5$ are hydrogen with an appropriate aldehyde or ketone.

The reduction process may be effected in conventional manner for the reduction of the carbonyl group of an amide to a methylene group, for example with diborane. Suitable reaction temperatures are from 0° to 100°. The acylation and the condensation processes may be effected in known manner.

The compounds of formula I may be isolated from the reaction mixture and purified in known manner. The compounds of formula I may exist in salt form. The free base forms may be converted into acid addition salt forms in the usual manner and vice versa. A suitable acid for salt formation is hydrochloric acid, hydrobromic acid or oxalic acid. When there is a hydroxy group present, the free form may be converted into an anionic salt form, such as a sodium salt.

The starting materials of formula II may be produced by reacting a compound of formula III

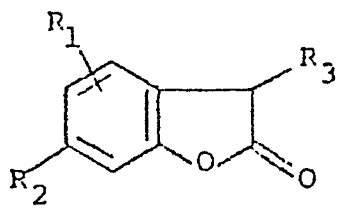

(III)

wherein $R_1$, $R_2$ and $R_3$ are as defined above, with an compound of formula IV

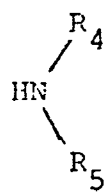

(IV)

wherein $R_4$ and $R_5$ are as defined above, and where required appropriately acylating any resulting phenol product and where required condensing any resulting primary amine product with an aldehyde or ketone.

The processes are conveniently effected in conventional manner if necessary protecting radicals which may interfere in the reaction or when selectivity is required.

Insofar as the production of any starting material is not particularly described, these are known or may be produced in conventional manner or in a manner analogous to that described above.

In the following non-limitative Examples all temperatures are indicated in degrees Centigrade.

EXAMPLE 1 : 2-(2-chloro-5-hydroxy-4-biphenylyl)ethylamine

184 ml of a solution of diborane (complexed with tetrahydrofuran) are added dropwise to a cooled solution of 14.8 g 2-(2-chloro-5-hydroxy-4-biphenylyl)-acetamide in 200 ml tetrahydrofuran. The reaction mixture is boiled for 16 hours and then poured onto ice/HCl. The aqueous phase is extracted with methylene chloride and the organic phases are combined, dried over $Na_2SO_4$ and evaporated. The residue is dissolved in ethyl acetate, ethereal HCl is added and the solution is evaporated, whereby the hydrochloride of the title compound is obtained. It melts at 206-207° after crystallization from ethanol/ether.

The starting material may be obtained as follows :

A solution of 17.5 g 5-chloro-2,3-dihydro-6-phenyl-benzofuran-2-one in tetrahydrofuran is added with stirring to 100 ml of a saturated aqueous ammonia solution. The reaction mixture is stirred at room temperature overnight and evaporated. The residue is evaporated with ethanol and toluene and dried under high vacuum to yield 2-(2-chloro-5-hydroxy-4-biphenylyl)acetamide (M.pt 123-124° after crystallization from ethyl acetate/ether).

From the appropriate compounds of

formula II, the following compounds of formula

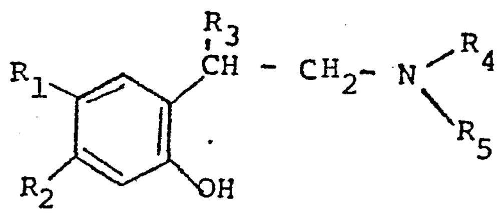

may be obtained in analogous manner to Example 1 :

| Ex. | $R_2$ | $R_1$ | $R_3$ | $R_4$ | $R_5$ | M.pt. 1) |
|---|---|---|---|---|---|---|
| 2 | phenyl | Cl | $CH_3$ | H | H | 266-267° 3) |
| 3 | cyclohexyl | Cl | $CH_3$ | H | H | 210-212° 3) |
| 4 | cyclohexyl | Cl | H | H | H | 205-206° 3) |
| 5 | cyclohexyl | $CH_3$ | H | H | H | - |
| 6 | phenyl | $CH_3$ | H | H | H | 218° 2) |
| 7 | phenyl | H | H | H | H | 140-142° |
| 8 | phenyl | H | $CH_3$ | H | H | 146-147° |
| 9 | cyclohexyl | H | $CH_3$ | H | H | 142-143° 3) |
| 10 | phenyl | Cl | H | $CH_3$ | $CH_3$ | 127° 3) |
| 11 | 4-Cl-phenyl | Cl | H | H | H | 178° 3) |
| 12 | 4-OH-phenyl | Cl | H | H | H | 202° 3) |
| 13 | 4-$CH_3$-phenyl | Cl | H | H | H | 234-236° 3) |
| 14 | 4-F-phenyl | Cl | H | H | H | 65° 4) 5) |
| 15 | 4-$NH_2$-phenyl | Cl | H | H | H | 300° 6) |
| 16 | phenyl | F | H | H | H | 248° 2) |
| 17 | cyclohexyl | H | H | H | H | 206-207° 3) |
| 18 | cyclohexyl | Cl | H | benzyl | H | 251-253° 3) |
| 19 | cyclohexyl | H | H | isopropyl | H | 206-207° 3) |
| 20 | cyclohexyl | Cl | H | $-(CH_2)_2-O(CH_2)_2-$ | | 142-143° |

1) free base unless otherwise specified

2) hydrobromide salt

3) hydrochloride

4) hydrogen oxalate

5) sintering point

6) dihydrobromide

EXAMPLE 22 : 2-(5-acetoxy-2-chloro-4-biphenylyl)ethylamine

4 g 2-(2-chloro-5-hydroxy-4-biphenylyl)ethylamine are dissolved in 30 ml trifluoroacetic acid with stirring and cooling and 1.5 ml of acetyl chloride are added dropwise within 2 minutes. The reaction mixture is stirred for 2 hours at room temperature and evaporated twice in the presence of ether. The residue is dissolved in ether and ethereal HCl is added until the mixture is at pH 2, whereupon the hydrochloride of the title compound is obtained. It melts at 185°.

EXAMPLE 23 : N-[2-(2-chloro-5-hydroxy-4-biphenylyl)-ethyl]-2-hydroxy-benzylidenamine

4 g 2-(2-chloro-5-hydroxy-4-biphenylyl) ethylamine, 1,8 ml salicylic aldehyde and 5 mg p-toluenesulfonic acid in 150 ml toluene are boiled for 3 hours. The reaction mixture is filtered and evaporated to yield the title compound. It melts at 145°.

The compounds of formula I are exhibit pharmacological activity in animals. In particular, the compounds of formula I inhibit oedemas and inflammations, as indicated in standard tests, for example by an inhibition of oedema formation in the carrageen paw oedema test in rats on p.o. administration of from 1 to 60 mg/kg animal body weight of the compounds.

The compounds are therefore indicated for use as anti-phlogistic agents.

Additionally, the compounds of formula I exhibit anti-arthritic activity, as indicated in standard tests, for example, by an inhibition of swelling in the Freund adjuvant arthritis latent period test in rats on p.o. administration of from 1 to 100 mg/kg animal body weight of the compounds.

The compounds are therefore further indicated for use as anti-arthritic agents.

Additionally, the compounds of formula I exhibit anti-pyretic activity, as indicated by a reduction of fever produced by yeast in the yeast-fever test on administration of 10 to 100 mg/kg p.o. of the compounds to rats.

The compounds are therefore further indicated for use as anti-pyretic agents. For all the above -mentioned uses an indicated daily dose is from about 100 to about 1000 mg, conveniently administered in divided doses 2 to 4 times a day in unit dosage form containing from about 25 to about 500 mg, or in sustained release form.

Additionally, the compounds of formula I exhibit analgesic activity as indicated in standard tests, for example, by an inhibition of the phenyl benzoquinone syndrome in mice on p.o. administration of from 3 to 50 mg/kg animal body weight of the compounds and in the Randall-Sellito test in rats on p.o. administration of from 3 to 100 mg/kg animal body weight of the compounds.

The compounds are therefore further indicated for use as analgesic agents. For this indication, an indicated daily dose is from about 100 to 350 mg, conveniently administered in divided doses 2 to 4 times a day in unit dosage form containing from about 25 to about 175 mg, or in sustained release form.

The compound of Example 1 is particularly interesting.

The compounds may be administered in pharmaceutically acceptable acid addition salt form. These salt forms exhibit the same order of activity as the free base forms.

The present invention also provides a pharmaceutical composition comprising a compound of formula I, in free base form or in pharmaceutically acceptable acid addition salt form in association with a pharmaceutically acceptable

diluent or carrier.

These compositions may be formulated in conventional manner so as to be for example a solution, a capsule or tablet.

PATENT CLAIMS

1. A compound of formula I

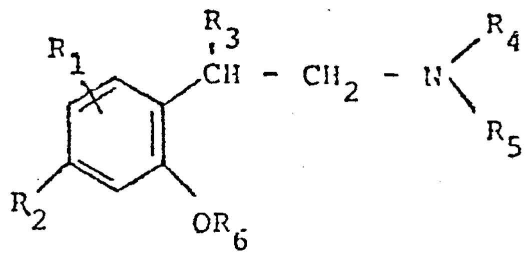

(I)

wherein

$R_1$ is hydrogen, halogen, hydroxy, nitro, $(C_{1-4})$alkyl, $(C_{1-4})$alkoxy, $(C_{1-4})$alkylthio, lower acyloxy or lower acylamino,

$R_2$ is $(C_{3-8})$cycloalkyl or a phenyl radical,

$R_3$ is hydrogen or $(C_{1-4})$alkyl,

$R_4$ and $R_5$ are the same or different and are hydrogen, $(C_{1-4})$alkyl or $(C_{7-11})$phenylalkyl, or

$R_4$ and $R_5$, together with the nitrogen atom to which they are bound, form a 1-pyrrolidinyl, piperidino, or morpholino radical, or

$R_4$ and $R_5$ together form a hydrolysable methylene moiety and

$R_6$ is hydrogen or a hydrolysable acyl radical

or an acid addition salt thereof.

2. A compound of claim 1 wherein $R_4$ and $R_5$ are the same or different and are hydrogen, $(C_{1-4})$alkyl or $(C_{7-11})$phenylalkyl, or $R_4$ and $R_5$, together with the nitrogen atom to which they are bound, form a 1-pyrrolidinyl, piperidino, or morpholino radical, and $R_6$ is hydrogen.

3. A compound of claim 2 wherein $R_1$ is para to the group $OR_6$ and $R_1$ is hydrogen, methyl or halogen, $R_2$ is cycloalkyl, phenyl or phenyl substituted by halogen, alkyl or alkoxy.

4. A compound of claim 3 wherein $R_2$ is phenyl.

5. A compound of claim 1 which is 2-(2-chloro-5-hydroxy-4-biphenylyl)ethylamine.

6. A process for the production of a compound of formula I which comprises reducing a compound of formula II

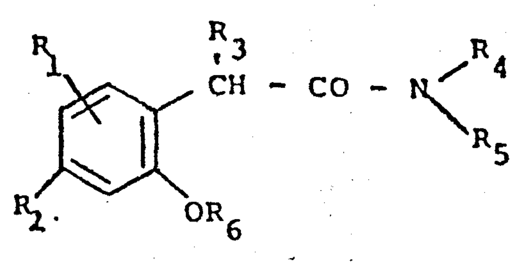

(II)

in which $R_1$ to $R_6$ are as defined in claim 1, and where required appropriately acylating any resulting product having a free hydroxy or amino group and/or condensing

any resulting compound wherein $R_4$ and $R_5$ are hydrogen with an appropriate aldehyde or ketone.

7. A pharmaceutical composition comprising a compound any one of claims 1 to 5 in free base form or in pharmaceutically acceptable acid addition salt form in association with a pharmaceutically acceptable diluent or carrier.

3700/RR/GR/JH

0006961

European Patent Office

# EUROPEAN SEARCH REPORT

Application number
[illegible]

## DOCUMENTS CONSIDERED TO BE RELEVANT

| Category | Citation of document with indication, where appropriate, of relevant passages | Relevant to claim |
|---|---|---|
| | CH-A- 299 155 (J.R. GEIGY)<br>* Patentanspruch 1 *<br>---- | 1,[illegible] |
| | CH-B- 202 664 (AKTIEBOLAGET [illegible])<br>* Patentanspruch *<br>---- | 1 |
| A | CH-A- 191 403 (F.HOFFMANN-LA ROCHE & CO.)<br>* Patentanspruch * | 1-3 |
| A | US-A- 2 795 613 ([illegible])<br>* Patentanspruch 2 *<br>---- | 1-3 |

**CLASSIFICATION OF THE APPLICATION (Int. Cl.[2])**

C 07 C 91/30
C 07 C 93/14
C 07 C [illegible]
A 61 K 31/135
C 07 C 93/2[illegible]

**TECHNICAL FIELDS SEARCHED (Int. Cl.[2])**

C 07 C 91/30
C 07 C 93/14
C 07 C 93/[illegible]

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

✓ The present search report has been drawn up for all claims

| Place of search | Date of completion of the search | Examiner |
|---|---|---|
| Wien | 2. August 1979 | [illegible] |

EPO Form 1503.1 06.78